# EUROPÄISCHE PATENTANMELDUNG

(11) **EP 1 985 237 A1**
(43) Veröffentlichungstag der Anmeldung: **29.10.2008**
(21) Anmeldenummer: 07008188.0
(22) Anmeldetag: 23.04.2007
(51) Int. Cl.: A61B 6/04, A61G 13/00, A61N 5/10

(54) **Einrichtung und Verfahren zur automatisierten Positionierung eines Patienten**

(71) Anmelder: Buck Engineering & Consulting GmbH, 72574 Bad Urach (DE)
(72) Erfinder: Buck, Matthias, 72764 Reutlingen (DE)
(74) Vertreter: Wasmuth, Rolf

(57) **Zusammenfassung**

Eine Einrichtung zur automatisierten Positionierung eines Patienten (13) umfasst ein bewegliches Patiententransportmittel (1, 51) mit einer Patientenaufnahme (2, 52) und eine Handhabungseinrichtung (7). Es ist eine Kopplungseinrichtung (29) zur Kopplung der Patientenaufnahme (2, 52) in einer Aufnahmeposition (18) mit der Handhabungseinrichtung (7) vorgesehen. Um eine gute Positionierung des Patienten zu ermöglichen, ist vorgesehen, dass die Einrichtung Positionierungsmittel zur Festlegung und/oder Bestimmung der Position des Patiententransportmittels (1, 51) relativ zu der Handhabungseinrichtung (7)in entkoppeltem Zustand von Patientenaufnahme (2, 52) und Handhabungseinrichtung (7) besitzt. Ein Verfahren zur automatisierten Positionierung eines Patienten (13) sieht vor, dass das Patiententransportmittel (1, 51) vor der Kopplung mit Positionierungsmitteln im Raum positioniert oder die Position des Patiententransportmittels (1, 51) mit den Positionierungsmitteln bestimmt wird.

## Beschreibung

Die Erfindung betrifft eine Einrichtung zur automatisierten Positionierung eines Patienten der im Oberbegriff des Anspruchs 1 angegebenen Gattung sowie ein Verfahren zur automatisierten Positionierung eines Patienten der im Oberbegriff des Anspruchs 13 angegebenen Gattung.

Aus der DE 10 2005 041 606 A1 ist eine Patientenpositioniervorrichtung bekannt, bei der ein Patientenhalterungsmodul mit einem Positionierarm bewegt wird. Der Positionierarm kann an das Patientenhalterungsmodul angekoppelt und von diesem abgekoppelt werden. Dabei ist vorgesehen, dass der Patient mit Hilfe einer Transportvorrichtung in den Bereich des Positionierarms gebracht und dort von dem Positionierarm aufgenommen wird.

Der Erfindung liegt die Aufgabe zugrunde, eine Einrichtung zur automatisierten Positionierung eines Patienten zu schaffen, die eine hohe Genauigkeit der Positionierung des Patienten ermöglicht. Eine weitere Aufgabe der Erfindung liegt darin, ein Verfahren zur automatisierten Positionierung eines Patienten anzugeben, mit dem der Patient mit einer hohen Genauigkeit positioniert werden kann. Diese Aufgabe wird durch eine Einrichtung zur automatisierten Positionierung eines Patienten mit den Merkmalen des Anspruchs 1 und durch ein Verfahren zur automatisierten Positionierung eines Patienten mit den Merkmalen des Anspruchs 13 gelöst.

Um den Patienten mit einer hohen Genauigkeit einer Behandlung zuführen zu können, muss die Kopplungseinrichtung eine geringe Toleranz aufweisen und die Patientenaufnahme in definierter Weise aufnehmen. Dies ist jedoch bei einer manuellen Positionierung des Patiententransportmittels, beispielsweise durch medizinisches Personal, nicht möglich, da sich hier die Positionierung nach Markierungen im Boden oder dgl. richtet, die lediglich eine grobe Vorpositionierung des Patiententransportmittels erreichen können.

Die Positionierungsmittel ermöglichen, die Position des Patiententransportmittels im Raum genau festzulegen oder zu bestimmen. Dadurch befindet sich das Patiententransportmittel in einer definierten Aufnahmeposition. Die Aufnahmeposition ist dabei bei einer Festlegung der Position durch das Positionierungsmittel für alle Patiententransportmittel gleich, während die Aufnahmeposition bei einer Bestimmung der Position von Patiententransportmittel zu Patiententransportmittel variieren kann. Da die Position bekannt ist, kann die Handhabungseinrichtung entsprechend gesteuert werden, und die Patientenaufnahme kann an der jeweiligen genauen Position aufgenommen werden. Dadurch ist es möglich, eine Kopplungseinrichtung mit geringen Toleranzen einzusetzen, so dass auch der Patient mit der Handhabungseinrichtung in eine genau definierte Behandlungsposition gebracht werden kann. Dadurch, dass die Position des Patiententransportmittels bekannt ist, kann der Ankoppelvorgang sanft ausgeführt werden. Eine Bewegung des Patienten zum Ausgleich von Toleranzen der Position des Patiententransportmittels kann weitgehend vermieden werden.

Für den Kopplungsvorgang ist es ausreichend, dass die Position des Patiententransportmittels festgelegt oder bestimmt wird. Um Redundanz und damit eine erhöhte Sicherheit zu erreichen, kann jedoch auch vorgesehen sein, die Position des Patiententransportmittels festzulegen und die festgelegte Position zusätzlich zu bestimmen, um sicherzustellen, dass sich das Patiententransportmittel tatsächlich in der gewünschten Position befindet.

Vorteilhaft umfassen die Positionierungsmittel mindestens eine Indexiereinrichtung zur Anordnung des Patiententransportmittels in der Aufnahmeposition. Die Indexiereinrichtung erlaubt auf einfache Weise eine Festlegung der Position des Patiententransportmittels. Vorteilhaft umfasst die Indexiereinrichtung mindestens einen Indexierdorn, der mit einer Indexieraufnahme an dem Patiententransportmittel zusammenwirkt. Der Indexierdorn ist dabei insbesondere in mindestens einer Richtung beweglich. Es kann jedoch auch vorgesehen sein, dass der Indexierdorn zum Ausgleich der Position des Patiententransportmittels in mehreren, insbesondere in allen drei Raumrichtungen beweglich ist. Um eine ungestörte Vorpositionierung des Patiententransportmittels zu erlauben, ist vorgesehen, dass die Indexiereinrichtung in Bodennähe angeordnet ist. Der Indexierdorn ist dabei insbesondere in vertikaler Richtung beweglich. Dabei können kleine Wege zur Positionierung ausreichend sein. Aufgrund der Indexieraufnahme wird das Patiententransportmittel auf dem Indexierdorn aufgrund der Schwerkraft positioniert. Es kann jedoch auch vorteilhaft sein, dass der Indexierdorn an dem Patiententransportmittel und die Indexieraufnahme im Raum angeordnet ist.

Um die Lage des Patiententransportmittels in allen drei Raumrichtungen und die Drehlage des Patiententransportmittels um alle drei Raumachsen festlegen zu können, sind insbesondere mindestens drei Indexiereinrichtungen vorgesehen, deren Indexierdorne mindestens in vertikaler Richtung beweglich sind. Dadurch kann eine genaue Positionierung des Patiententransportmittels unabhängig von Bodenunebenheiten oder von einer Abnutzung der Rollen des Patiententransportmittels erreicht werden. Das Patiententransportmittel kann von der Indexiereinrichtung vom Boden abgehoben werden.

Vorteilhaft umfassen die Positionierungsmittel ein optisches System mit einer optischen Marke an dem Patiententransportmittel und einem optischen Erfassungssystem zur Erfassung der Position der optischen Marke. Das optische System erlaubt eine berührungslose Erfassung der Position des Patiententransportmittels. Dadurch kann die Position des Patiententransportmittels einfach und genau bestimmt werden. Es kann jedoch auch vorgesehen sein; dass die Positionierungsmittel ein Funksystem mit einer Funkmarke an dem Patiententransportmittel und einer Funkeinrichtung zur Erfassung der Position der Funkmarke umfassen. Die Funkmarke kann dabei als Positionssensor dienen, dessen Position beispielsweise über ein globales Positionierungssystem (GPS) oder, um eine höhere Genauigkeit zu erreichen, über ein lokales Positionierungssystem bestimmt werden kann. Dabei kann die Funkmarke auch so ausgebildet sein, dass nicht nur die Position, sondern auch die Ausrichtung des Patiententransportmittels bestimmt werden kann. Dazu können auch mehrere Marken an dem Patiententransportmittel vorgesehen sein. Das Erfassungssystem ist insbesondere fest im Raum angeordnet. Es kann jedoch auch vorgesehen sein, dass das Erfassungssystem an der Handhabungseinrichtung angeordnet ist und mit der Handhabungseinrichtung im Raum bewegbar ist. Dadurch können fest installierte Einrichtungen im Raum entfallen. Allerdings ist das Erfassungssystem bei der Anordnung an der Handhabungseinrichtung im Betrieb der Strahlung ausgesetzt, mit der der Patient behandelt wird. Dies kann zu einer Verkürzung der Lebensdauer des Erfassungssystems führen.

Sowohl die optische Marke als auch die Funkmarke geben die Position des Patiententransportmittels vorteilhaft in allen drei Raumrichtungen und die Drehlage des Patiententransportmittels um alle drei Raumachsen an.

Vorteilhaft umfasst die Einrichtung mehrere Patiententransportmittel, wobei sich die Marken der einzelnen Patiententransportmittel zur Identifizierung der einzelnen Patiententransportmittel unterscheiden. Dadurch ist es möglich, Eigenschaften eines einzelnen Patiententransportmittels zu berücksichtigen, beispielsweise spezifische Verformungen aufgrund von Belastungen im Betrieb oder spezifische geometrische Eigenschaften der einzelnen Patiententransportmittel, die sich im Laufe der Zeit aufgrund von Alterungsvorgängen ändern können. Dadurch kann die Genauigkeit der Positionierung des Patienten erhöht werden.

Vorteilhaft umfassen die Positionierungsmittel eine Vorpositionierungseinrichtung zur Vorpositionierung des Patiententransportmittels im Raum. Die Vorpositionierungseinrichtung kann beispielsweise als Anschlag, Bodenwelle oder Markierung ausgebildet sein. Auch andere Ausbildungen der Vorpositionierungseinrichtung können vorteilhaft sein.

Vorteilhaft ist mindestens eine Fixiereinrichtung zur Fixierung des Patiententransportmittels vorgesehen. Die Fixiereinrichtung stellt sicher, dass die Position des Patiententransportmittels nach der Anordnung des Patiententransportmittels in der definierten Aufnahmeposition oder nach der Bestimmung der Position des Patiententransportmittels nicht mehr verändert wird. Die Fixiereinrichtung ist dabei vorteilhaft so ausgelegt, dass die Position des Patiententransportmittels auch von der Handhabungseinrichtung nicht verändert werden kann. Insbesondere ist die Patientenaufnahme des Patiententransportmittels fixiert. Dabei können auch mehrere Fixiereinrichtungen vorgesehen sein.

Vorteilhaft ist eine Positionsüberwachungseinrichtung zur Überwachung der Anordnung des Patiententransportmittels in der Aufnahmeposition vorgesehen. Die Positionsüberwachungseinrichtung stellt sicher, dass das Patiententransportmittel in der definierten bzw. der bestimmten Aufnahmeposition angeordnet ist. Vorteilhaft umfasst die Positionsüberwachungseinrichtung mindestens einen Sensor, der insbesondere als Drucksensor gestaltet ist. Es kann jedoch auch vorgesehen sein, dass die Positionsüberwachungseinrichtung mindestens einen Schalter, insbesondere einen induktiven Schalter oder einen elektrischen Kontakt umfasst.

Vorteilhaft sind Überwachungsmittel zur Überwachung der Kopplung der Patientenaufnahme mit der Handhabungseinrichtung vorgesehen. Die Überwachungsmittel stellen sicher, dass eine sichere Kopplung der Patientenaufnahme an die Handhabungseinrichtung erfolgt. Die Überwachungsmittel umfassen vorteilhaft Mittel zur Überwachung des Motorstroms mindestens eines Antriebsmotors der Handhabungseinrichtung. Damit kann ermittelt werden, ob die Handhabungseinrichtung beispielsweise an Hindernissen anliegt oder ob sich das Patiententransportmittel in der definierten Aufnahmeposition befindet. Vorteilhaft umfassen die Überwachungsmittel mindestens einen Sensor, der die Kopplung überwacht.

Um ein weiches, für den Patienten angenehmes Ankoppeln der Patientenaufnahme an die Handhabungseinrichtung zu erreichen, ist vorgesehen, dass die Kopplungseinrichtung eine Toleranzausgleichseinrichtung zum Ausgleich von Toleranzen zwischen Handhabungseinrichtung und Patientenaufnahme aufweist. Eine derartige Toleranzausgleichseinrichtung kann beispielsweise ein Nullpunktspannsystem sein. Als Nullpunktspannsystem kann beispielsweise eine Fügehilfe vorgesehen sein, die konisch ausgebildet ist und die den zurückzulegenden Relativweg von Patientenaufnahme und Handhabungseinrichtung vorgibt. Dies kann beispielsweise durch eine Kraft-Momentenregelung über Motorströme oder einen entsprechenden Sensor ergänzt werden. Das Nullpunktspannsystem kann dabei insbesondere nur die durch das System selbst zugelassenen Toleranzen ausgleichen. Es kann auch vorgesehen sein, dass eine der Komponenten der Kopplungseinrichtung schwimmend gelagert ist, beispielsweise in den beiden horizontalen Richtungen, und dass die Komponenten dann relativ zueinander positioniert werden.

Vorteilhaft ist die Handhabungseinrichtung ein Roboterarm, der mindestens eine vertikale Bewegungsachse und mindestens zwei horizontale Bewegungsachsen aufweist. Es kann jedoch auch vorgesehen sein, dass der Roboterarm weitere Bewegungsachsen aufweist, um weitere Freiheitsgrade der Bewegung zu erreichen.

Für ein Verfahren zur automatisierten Positionierung eines Patienten, mit einer Einrichtung zur automatisierten Positionierung des Patienten, wobei die Einrichtung ein bewegliches Patiententransportmittel mit einer Patientenaufnahme und eine Handhabungseinrichtung umfasst, und wobei die Patientenaufnahme mit einer Kopplungseinrichtung mit der Handhabungseinrichtung gekoppelt wird, ist vorgesehen, dass das Patiententransportmittel vor der Kopplung mit Positionierungsmitteln im Raum positioniert und/oder die Position des Patiententransportmittels mit den Positionierungsmitteln bestimmt wird.

Dadurch kann eine Kopplung der Patientenaufnahme an die Handhabungseinrichtung mit hoher Genauigkeit erreicht werden. Für die Positionierung des Patiententransportmittels ist dabei insbesondere vorgesehen, dass das Patiententransportmittel positioniert wird und das Erreichen der Position von Überwachungsmitteln überwacht wird, dass nach dem Erreichen der Position das Erreichen der Position an eine Steuerung gemeldet wird, und dass die Steuerung die Handhabungseinrichtung zu der Aufnahmeposition des Patiententransportmittels bewegt und die Patientenaufnahme mit der Handhabungseinrichtung koppelt. Für die Bestimmung der Position des Patiententransportmittels ist insbesondere vorgesehen, dass die Position des Patiententransportmittels, das insbesondere im Raum vorpositioniert ist, bestimmt wird, anschließend die bestimmte Position an eine Steuerungseinrichtung übermittelt wird und die Steuerungseinrichtung die Handhabungseinrichtung zu der von den Positionierungsmitteln übermittelten Position bewegt und die Handhabungseinrichtung mit der Patientenaufnahme koppelt.

Vorteilhaft wird die Patientenaufnahme vor der Kopplung von einer Fixiereinrichtung fixiert. Dadurch ist sichergestellt, dass sich die Patientenaufnahme beim Ankoppelvorgang nicht bewegen kann. Vorteilhaft wird die Patientenaufnahme nach der Kopplung an die Handhabungseinrichtung von der Fixiereinrichtung freigegeben. Dadurch kann die Patientenaufnahme mit der Handhabungseinrichtung bewegt und der Patient in die vorgegebene Position gebracht werden. Es ist vorgesehen, dass die Handhabungseinrichtung Mittel zur Erfassung der im Betrieb an der Handhabungseinrichtung wirkenden Kräfte aufweist, und dass die an der Handhabungseinrichtung wirkenden Kräfte überwacht werden. Die Überwachung der Kräfte kann dabei beispielsweise über die Überwachung von Motorströmen der Antriebsmotoren der Handhabungseinrichtung oder über zusätzliche Sensoren erfolgen. Aufgrund der Überwachung der wirkenden Kräfte kann sichergestellt werden, dass die Patientenaufnahme an die Handhabungseinrichtung angekoppelt wird. Die Sicherheit des Systems wird erhöht.

Vorteilhaft weist das Patiententransportmittel mindestens eine Marke auf, wobei das Patiententransportmittel anhand der Marke identifiziert wird, und wobei die Behandlungsposition des Patienten mit gespeicherten geometrischen Daten des identifizierten Patiententransportmittels korrigiert wird. Durch die Identifizierung des Patiententransportmittels und die Korrektur der Behandlungsposition des Patienten mit den gespeicherten geometrischen Daten des identifizierten Patiententransportmittels kann die Genauigkeit der Positionierung des Patienten noch erhöht werden. Alterungsvorgänge, Abnutzung oder Verformungen aufgrund des Gewichts des Patienten können berücksichtigt werden. Um die gespeicherten geometrischen Daten zu aktualisieren, ist vorgesehen, dass die Position des Patienten bei der Behandlung über ein bildgebendes Verfahren ermittelt oder überprüft wird, und dass die gespeicherten geometrischen Daten des Patiententransportmittels anhand der tatsächlichen Position des . Patienten korrigiert werden. Dadurch lässt sich eine hohe Genauigkeit der Positionierung erreichen, ohne dass bei jedem Patienten die Überprüfung der Position über ein bildgebendes Verfahren, beispielsweise über ein Röntgenverfahren oder dgl. notwendig ist.

Ausführungsbeispiele der Erfindung werden im Folgenden anhand der Zeichnung erläutert. Es zeigen:
- Fig. 1: eine perspektivische, schematische Darstellung einer Einrichtung zur automatisierten Positionierung eines Patienten,
- Fig. 2: das Patiententransportmittel der Einrichtung aus Fig. 1 in perspektivischer Darstellung,
- Fig. 3 und Fig. 4: schematische Darstellungen des Patiententransportmittels aus Fig. 2 in perspektivischen Seitenansichten,
- Fig. 5: eine Seitenansicht des Bodens im Bereich der Rolle des Patiententransportmittels,
- Fig. 6: eine Seitenansicht der Behandlungsposition der Einrichtung aus Fig. 1 in schematischer Darstellung,
- Fig. 7: eine perspektivische Darstellung der Patientenliege des Patiententransportmittels aus Fig. 1,
- Fig. 8: eine Ansicht von unten auf die Patientenliege aus Fig. 7,
- Fig. 9: eine Seitenansicht der Patientenliege in Richtung des Pfeils IX in Fig. 8,
- Fig. 10: eine schematische Darstellung der Koppelaufnahme der Handhabungseinrichtung,
- Fig. 11: ein Ausführungsbeispiel eines Patiententransportmittels in Aufnahmeposition,
- Fig. 12: das Patiententransportmittel aus Fig. 11 beim Transport des Patientenstuhls,
- Fig. 13: eine schematische Darstellung des Ablaufs eines Verfahrens zur automatisierten Positionierung eines Patienten.

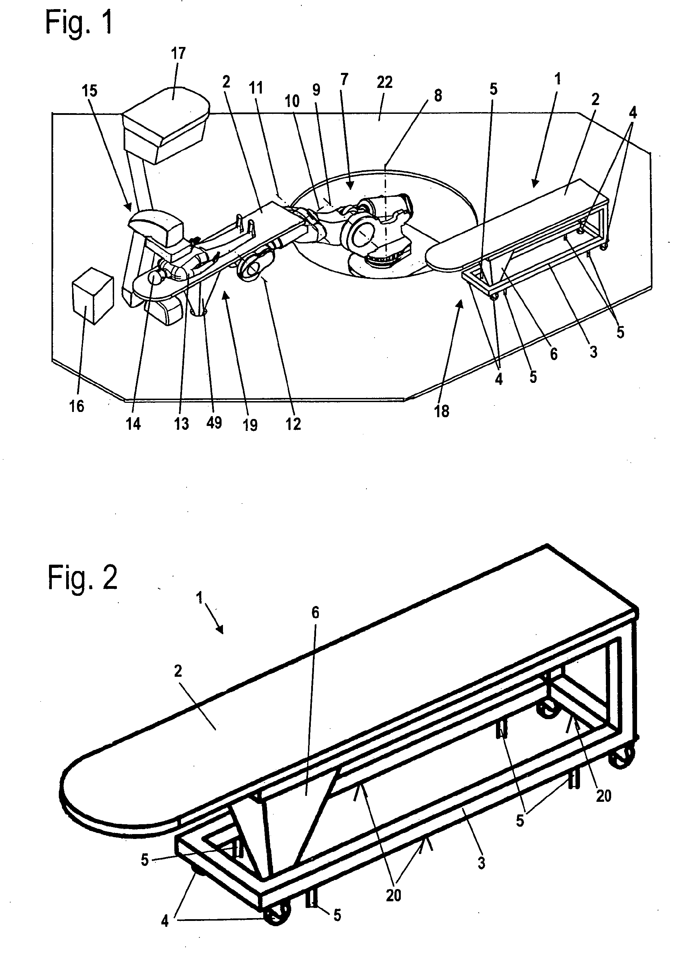

Fig. 1 zeigt in schematischer perspektivischer Darstellung eine Einrichtung zur automatisierten Positionierung und Behandlung eines Patienten. Die Einrichtung besitzt einen Boden 22, der beispielsweise der Boden eines Behandlungsraums ist und in dem versenkt eine Handhabungseinrichtung 7 angeordnet ist. Die Handhabungseinrichtung 7 kann jedoch auch auf dem Boden 22 angeordnet sein. Die Handhabungseinrichtung 7 dient zur Aufnahme einer Patientenliege 2 in einer Aufnahmeposition 18 und zur Positionierung der Patientenliege 2 in einer Behandlungsposition 19. Zur Verdeutlichung sind in Fig. 1 beide Positionen der Patientenliege 2 gezeigt.

Im Betrieb wird die Patientenliege 2 mit dem darauf fixierten Patienten 13 in der Aufnahmeposition 18 aufgenommen, zur Behandlungsposition 19 bewegt und nach der Behandlung wieder in der Aufnahmeposition 18 angeordnet. In der Behandlungsposition 19 kann der Patient 13 einer Diagnoseeinrichtung 15, beispielsweise einer Röntgeneinrichtung, einem Kernspintomografen oder einem anderen bildgebenden Verfahren zugeführt werden. Im Ausführungsbeispiel ist die Diagnoseeinrichtung 15 mit einer Deckenhalterung 17 an der Decke fixiert. Der Patient 13 kann zusätzlich oder alternativ einer Therapieeinrichtung 16 zugeführt werden. Die Therapieeinrichtung 16 kann ebenfalls eine Röntgeneinrichtung sein. Vorteilhaft ist die Therapieeinrichtung 16 eine Einrichtung zur Strahlentherapie, bei der der Patient insbesondere mit Ionenstrahlen bestrahlt wird. Auch andere Therapieeinrichtungen 16 können vorgesehen sein. Um sicherzustellen, dass sich der Patient 13 auf der Patientenliege 2 nicht bewegt, ist eine Patientenfixierung 14 vorgesehen.

Der Patient wird vor der Behandlung mit einem Patiententransportmittel 1 in den Behandlungsraum gebracht. Das Patiententransportmittel 1 umfasst die Patientenliege 2, die die Patientenaufnahme bildet, sowie ein Fahrgestell 3, auf dem die Patientenliege 2 angeordnet ist. Das Fahrgestell 3 besitzt im Ausführungsbeispiel vier Rollen 4, mit denen das Fahrgestell 3 über den Boden bewegt werden kann. Das Patiententransportmittel 1 wird mit dem Patienten 13 zunächst im Behandlungsraum vorpositioniert. Anschließend erfolgt eine Positionierung des Patiententransportmittels 1 oder eine Bestimmung der Position des Patiententransportmittels 1. Dies wird im Folgenden noch näher erläutert. Anschließend wird das Fahrgestell 3 des Patiententransportmittels 1 über Fixierbolzen 5 im Raum fixiert. Ein optisches System ermittelt und überwacht die Position der Patientenliege 2. Hierzu ist in Fig. 1 der Strahlengang 6 des optischen Systems gezeigt.

Die Handhabungseinrichtung 7 ist als Roboterarm ausgebildet, der mehrere Bewegungsachsen aufweist, um eine freie Positionierung der Patientenliege 2 im Raum in der Behandlungsposition 19 zu ermöglichen. Die Handhabungseinrichtung 7 besitzt eine vertikale Bewegungsachse 8, eine erste horizontale Bewegungsachse 9, eine zweite horizontale Bewegungsachse 10, die senkrecht zur ersten horizontalen Bewegungsachse 9 und zur vertikalen Bewegungsachse 8 steht, sowie eine dritte horizontale Bewegungsachse 11 und eine vierte horizontale Bewegungsachse 12, die beide parallel zur ersten horizontalen Bewegungsachse 9 verlaufen. In der Behandlungsposition 19 ist ein weiteres optisches System zur Erfassung und Überwachung der Position der Patientenliege 2 angeordnet. Dies ist über den Strahlengang 49 angedeutet.

Fig. 2 zeigt das Patiententransportmittel 1 in vergrößerter Darstellung. Wie Fig. 2 zeigt, sind vier Fixierbolzen 5 vorgesehen, die das Gestell 3 fixieren. Außerdem sind drei Indexierdorne 20 einer Indexiereinrichtung vorgesehen. Die Indexiereinrichtung positioniert das Patiententransportmittel 1, also das Fahrgestell 3 mit der Patientenliege 2, in der definierten Aufnahmeposition 18. Dadurch, dass drei Indexierdorne 20 vorgesehen sind, kann die Position des Patiententransportmittels 1 in allen drei Raumrichtungen und allen drei Raumachsen festgelegt werden. Um Toleranzen der Vorpositionierung zwischen dem Patiententransportmittel 1 und den Indexierdornen 20 auszugleichen, kann vorgesehen sein, dass die Indexierdorne 20 parallel zum Boden 22, also in den beiden senkrecht zum Doppelpfeil 28 stehenden Richtungen, ebenfalls beweglich sind.

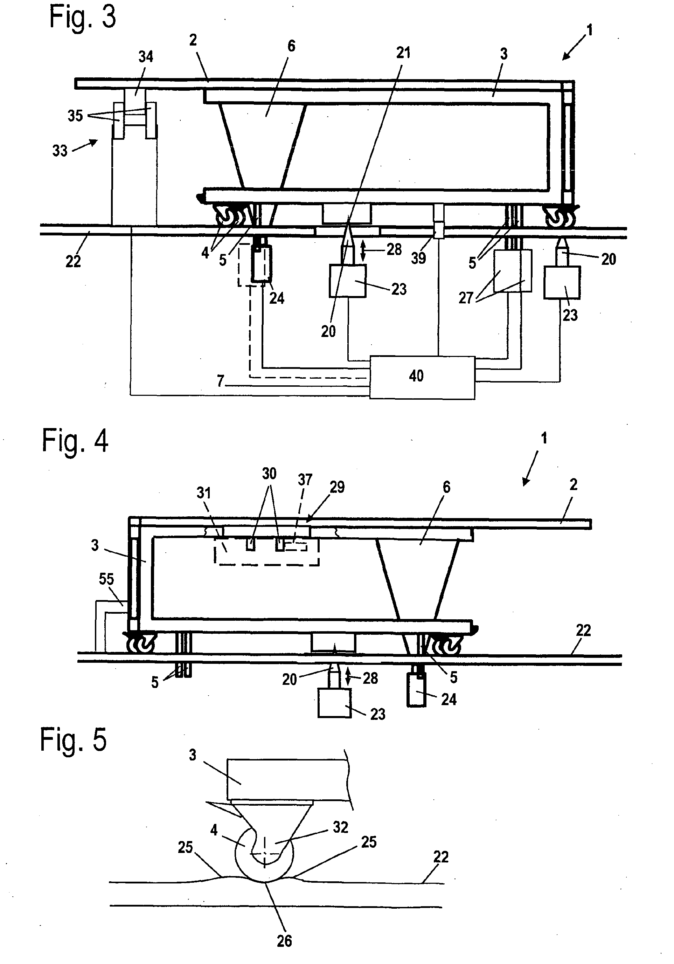

In Fig. 3 sind die Indexiereinrichtungen schematisch gezeigt. Jede Indexiereinrichtung besitzt eine Antriebseinrichtung 23 zur Bewegung des Indexierdorns 20 in vertikaler Richtung. Die vertikale Richtung ist durch den Doppelpfeil 28 angedeutet. Die vertikale Richtung verläuft senkrecht zum Boden 22 in Wirkrichtung der Schwerkraft. Am Fahrgestell 3 ist für jede Indexiereinrichtung eine Indexieraufnahme 21 vorgesehen. Die Indexieraufnahme 21 und der Indexierdorn 20 verlaufen konisch, so dass sich eine automatische Zentrierung ergibt. Um die Patientenliege 2 auch in der Höhe genau positionieren zu können, ist vorgesehen, dass die Indexierdorne 20 das Patiententransportmittel 1 anheben. Dadurch werden Unebenheiten des Bodens oder Abnutzungen der Rollen 4 ausgeglichen.

Die vier Fixierbolzen 5 besitzen jeweils eine Antriebseinrichtung 27, die die Fixierbolzen 5 in vertikaler Richtung bewegt. Die Fixierbolzen 5 ragen in die zugeordneten, nicht gezeigten Aufnahmen des Fahrgestells 3 und fixieren das Fahrgestell 3 dadurch. Zusätzlich ist eine Fixiereinrichtung 33 für die Patientenliege 2 vorgesehen. Die Fixiereinrichtung 33 besitzt zwei Greifbacken 35, die fest im Behandlungsraum installiert sind. Die beiden Greifbacken 35 wirken mit einem Greifabschnitt 34 an der Patientenliege 2 zusammen und fixieren die Patientenliege 2 an dem Greifabschnitt 34.

Wie Fig. 3 zeigt, sind die Indexiereinrichtungen und die Fixierbolzen 5 im Boden 22 versenkt angeordnet. Die Indexieraufnahmen 21 sind am Fahrgestell 3 vorteilhaft bodennah angeordnet, so dass nur ein geringer Weg der Indexierdorne 20 notwendig ist. Hier können bereits einige Millimeter, beispielsweise etwa 5 mm, ausreichend sein. Im Boden 22 versenkt ist außerdem eine Positionsüberwachungseinrichtung 39 angeordnet, die die Position des Patiententransportmittels 1, nämlich des Fahrgestells 3 in der Aufnahmeposition 18 überwacht. Die Positionsüberwachungseinrichtung 39 ist insbesondere ein Sensor, beispielsweise ein Drucksensor. Die Positionsüberwachungseinrichtung 39 kann jedoch auch ein Schalter, beispielsweise ein induktiver Schalter oder ein elektrischer Kontakt, sein. Es können auch mehrere Positions-überwachungseinrichtungen 39 vorgesehen sein, die auch unterschiedlich ausgestaltet sein können.

Zur Erfassung und Überwachung der Position der Patientenliege 2 ist ein optisches Erfassungssystem 24 vorgesehen, das ebenfalls im Boden 22 versenkt angeordnet ist. Das optische Erfassungssystem 24 kann beispielsweise ein Kamerasystem oder dgl. sein, das den Strahlengang 6 erzeugt. Die Strahlen, die den Strahlengang 6 erzeugen, können dabei sichtbare oder unsichtbare Strahlung sein. Die Fixiereinrichtung 33, die Antriebseinrichtungen 27 der Fixierbolzen 5, die Antriebseinrichtungen 23 der Indexierdorne 20 und die Positionsüberwachungseinrichtung 39 sind mit einer Steuerung 40 verbunden. Die Steuerung 40 überwacht die Signale der Erfassungs- und Überwachungssysteme und steuert die Aktoren entsprechend an. Die Steuerung 40 ist auch mit der Handhabungseinrichtung 7 verbunden.

Fig. 4 zeigt das Patiententransportmittel 1 von der anderen Seite. Zur Vereinfachung sind nicht alle Einrichtungen gezeigt. Es ist eine Kopplungseinrichtung 29 vorgesehen, die von zwei an der Patientenliege 2 fixierten Koppelbolzen 30 und einer in Fig. 4 gestrichelt gezeigten Koppelaufnahme 31 gebildet ist. Die Koppelaufnahme 31 ist fest an der Handhabungseinrichtung 7 angeordnet und wird von der Handhabungseinrichtung 7 bewegt. Zur Überwachung des Kopplungsvorgangs ist an der Koppelaufnahme 31 mindestens ein Sensor 37 vorgesehen, der überwacht, ob einer oder beide der Koppelbolzen 30 in der Koppelaufnahme 31 angeordnet sind. Die Koppelbolzen 30, die mit der Koppelaufnahme 31 zusammenwirken, sind lediglich ein Ausführungsbeispiel einer Kopplungseinrichtung 29. Es können auch andere Kopplungseinrichtungen 29 vorgesehen sein.

Zur Vorpositionierung des Patiententransportmittels 1 ist in Fig. 4 schematisch ein Anschlag 55 gezeigt, der fest mit dem Boden 22 verbunden ist. Zur Vorpositionierung können auch andere Einrichtungen im Raum vorgesehen sein wie beispielsweise Markierungen oder dgl..

Fig. 5 zeigt ein weiteres Ausführungsbeispiel einer Einrichtung zur Vorpositionierung. Hier sind im Boden 22 zwei Bodenwellen 25 vorgesehen, zwischen denen eine Vertiefung 26 gebildet ist, in der eine Rolle 4 angeordnet ist. Die Rolle 4 ist über eine Lagerung 32 mit dem Fahrgestell 3 verbunden. Es können auch für mehrere oder alle Rollen 4 Vertiefungen 26 im Boden 22 vorgesehen sein.

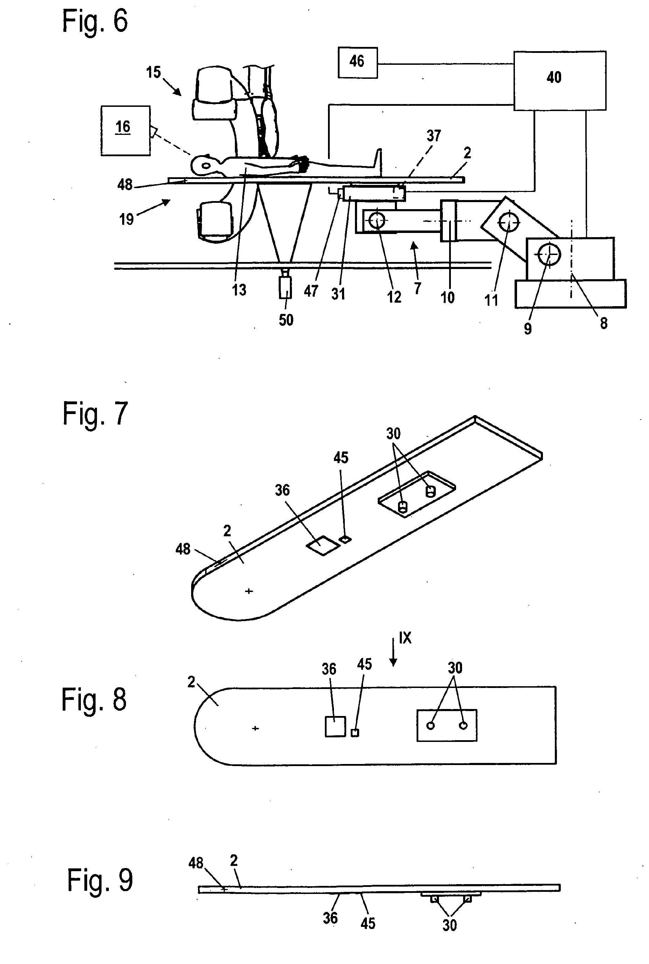

Fig. 6 zeigt die Patientenliege 2 in der Behandlungsposition 19. Die Handhabungseinrichtung 7 positioniert die Patientenliege 2 in der Behandlungsposition 19. Die Patientenliege 2 ist im Bereich des zu behandelnden Kopfs des Patienten 13 mit einer Röntgenmarke 48 versehen. Statt einer Röntgenmarke 48 kann auch eine andere in einem bildgebenden Verfahren gut zu erkennende Marke vorgesehen sein.

Wird mit der Diagnoseeinrichtung 15 eine Abbildung des Patienten 13 im Bereich seines Kopfes erstellt, beispielsweise eine Röntgenaufnahme, so ist auf dieser Aufnahme auch die Röntgenmarke 48 zu erkennen. Die Position des Patienten kann dadurch korrigiert werden. Über die Position der Röntgenmarke 48 im Raum ist auch die relative Position der Röntgenmarke 48 zur Koppelaufnahme 31 der Handhabungseinrichtung 7 bekannt. Dieser Abstand kann sich im Laufe der Zeit beispielsweise aufgrund von Alterungsvorgängen an der Patientenliege 2 ändern. Auch aufgrund von Toleranzen in der Fertigung der Patientenliegen 2 können sich geometrische Abweichungen zwischen den einzelnen Patientenliegen 2 ergeben. Diese Abweichungen werden mit der Röntgenmarke 48 erkannt und können bei der Positionierung berücksichtigt werden.

Wie Fig. 6 zeigt, ist in der Behandlungsposition 19 das optische Erfassungssystem 50 vorgesehen, das die Position der Patientenliege 2 überwacht. Zusätzlich kann die in den Figuren 7 bis 9 gezeigte Funkmarke 45 an der Patientenliege 2 vorgesehen sein. Wie Fig. 6 zeigt, ist im Behandlungsraum eine Funkeinrichtung 46 vorgesehen, die die Position der Funkmarke 45 erfasst. Es kann auch nur eine der Marken 36, 45 und 48 oder eine Kombination von zwei der Marken vorgesehen sein. Auch weitere Marken zur Überwachung und Sicherung der Position der Patientenliege 2 können zweckmäßig sein. Auch die Marken 36 und 45 erlauben die Überwachung und Berücksichtigung von geometrischen Abweichungen der Patientenliege 2. Es ist vorgesehen, dass die optische Marke 36 und/oder die Funkmarke 45 sich von Patientenliege 2 zur Patientenliege 2 unterscheiden. Dadurch können zur Positionierung des Patienten 13 die individuellen Geometriedaten jeder einzelnen Patientenliege 2 herangezogen werden.

Es ist vorgesehen, dass die in den Figuren 7 bis 9 gezeigte optische Marke 36 so ausgebildet ist, dass sowohl die Anordnung der Patientenliege 2 in allen drei Raumrichtungen als auch die Ausrichtung der Patientenliege 2 bezüglich allen drei Raumachsen möglich ist, so dass auch ein Kippen der Patientenliege 2 erkannt wird. Dadurch, dass die Position der Patientenliege 2 mit mehreren Systemen im Betrieb überwacht und ermittelt wird, ist die Sicherheit erhöht.

Wie Fig. 6 zeigt, ist an der Koppelaufnahme 31 ein Sensor 47 angeordnet, der ebenfalls mit der Steuerung 40 verbunden ist. Auch die Funkeinrichtung 46 ist mit der Steuerung 40 verbunden. Über den Sensor 47 kann der Koppelvorgang überwacht werden. Zur Bewegung der Handhabungseinrichtung 7 sind üblicherweise Antriebsmotoren vorgesehen, insbesondere Elektromotoren. Zur Überwachung des Kopplungsvorgangs können die Motorströme der Antriebsmotoren überwacht werden. Bei einem Stromanstieg liegt die Handhabungseinrichtung an einem Hindernis an. Dadurch kann die Sicherheit bei der Positionierung des Patienten 13 noch weiter erhöht werden.

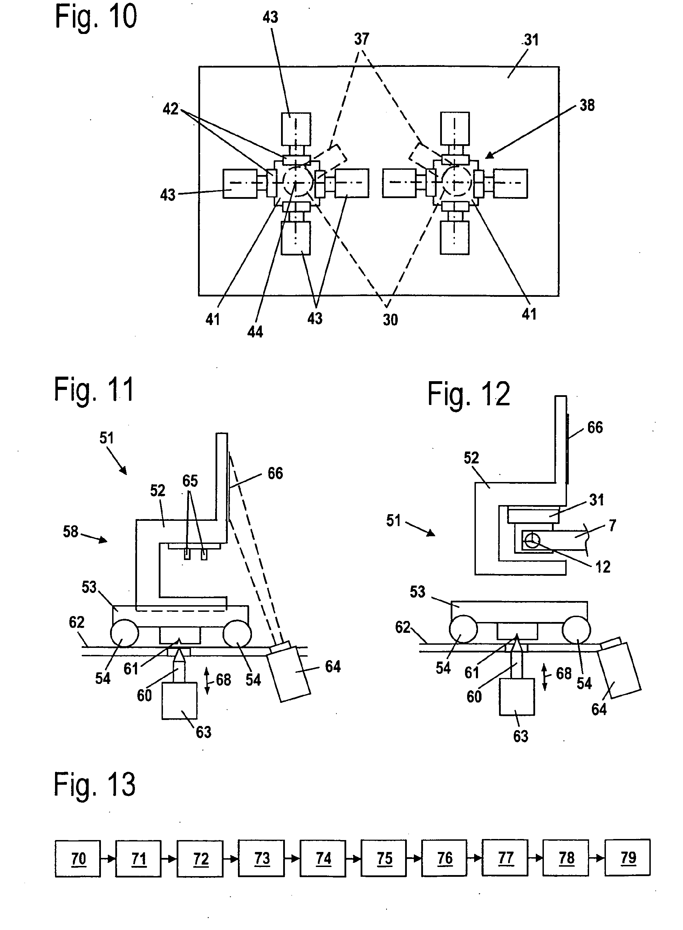

Um den Kopplungsvorgang weich und für den Patienten angenehm zu gestalten, ist die in Fig. 10 gezeigte Toleranzausgleichseinrichtung 38 zum Ausgleich von Toleranzen zwischen der Patientenliege 2 und der Handhabungseinrichtung 7 vorgesehen. In Fig. 10 sind die beiden Koppelbolzen 30 der Patientenliege 2 gestrichelt gezeigt. Die beiden Koppelbolzen 30 sind nicht zentriert zu Mittelachsen 44 der Aufnahmen 41 für die Koppelbolzen 30 angeordnet, sondern außermittig. Die Aufnahmen 41 sind von insgesamt vier Zylindern 43 begrenzt, die jeweils von einem Kolben 42 betätigt werden. Beim Koppelvorgang werden die Zylinder 43 zunächst nach außen gefahren, so dass die Koppelbolzen 30 auch dann in den Aufnahmen 41 angeordnet werden können, wenn die Koppelbolzen 30 nicht zentrisch zu den Mittelachsen 44 der Aufnahmen 41 angeordnet sind. Anschließend werden die Kolben 42 von den Zylindern 43 nach innen bewegt. Dadurch werden die Koppelbolzen 30 in den Aufnahmen 41 zentriert. Beim Koppelvorgang kann eine Überwachung der auftretenden Kräfte an den Kolben 42 vorgesehen sein, um sicherzustellen, dass die Koppelbolzen 30 korrekt in den Aufnahmen 41 angeordnet sind.

Die Figuren 11 und 12 zeigen ein Ausführungsbeispiel eines Patiententransportmittels 51. Das Patiententransportmittel 51 umfasst einen Patientenstuhl 52, der die Patientenaufnahme bildet und auf dem der Patient, der in den Figuren 11 und 12 nicht gezeigt ist, sitzend angeordnet und fixiert werden kann. Dies kann für spezielle Behandlungen vorteilhaft sein. In Fig. 11 ist das Patiententransportmittel 51 in einer Aufnahmeposition 58 gezeigt. Der Patientenstuhl 52 ist auf einem Fahrgestell 53 angeordnet, das zum Führen über den Boden vier Rollen 54 aufweist. An der dem Boden zugewandten Seite des Fahrgestells 53 ist benachbart zum Boden eine Indexieraufnahme 61 angeordnet, die mit einem Indexierdorn 60 zusammenwirken kann. Der Indexierdorn 60 besitzt eine Antriebseinrichtung 63, die den im Boden 62 versenkt angeordneten Indexierdorn 60 in vertikaler Richtung in Richtung des in Fig. 11 gezeigten Doppelpfeils 68 bewegen kann.

Am Patientenstuhl 52 ist im Bereich der Lehne eine optische Marke 66 angeordnet, die von einem ebenfalls im Boden 62 angeordneten optischen Erfassungssystem 64 erfasst werden kann. Das optische Erfassungssystem 64 erfasst dabei vorteilhaft die Anordnung der optischen Marke 66 in allen drei Raumrichtungen und um alle drei Raumachsen. Vorteilhaft ist über die optische Marke 66 zusätzlich eine Identifizierung des Patientenstuhls 52 möglich. Hierzu besitzt jedes Patiententransportmittel 51, also jeder Patientenstuhl 52, eine unterschiedliche optische Marke 66, die von dem optischen Erfassungssystem 64 erkannt wird. An dem Patientenstuhl 52 ist unterhalb der Sitzfläche ein Koppelelement vorgesehen, das durch zwei Koppelbolzen 65 gebildet ist.

Fig. 12 zeigt das Patiententransportmittel 51 nach dem Abheben des Patientenstuhls 52. An den beiden Koppelbolzen 65 ist die Koppelaufnahme 31 der Handhabungseinrichtung 7 angekoppelt. Vor dem Ankoppeln wurde die Position des Patiententransportmittels 51 über den Indexierdorn 60 festgelegt. Hierzu wurde der Indexierdorn 60 von der Antriebseinrichtung 63 in Richtung des Doppelpfeils 68 nach oben in die Indexieraufnahme 61 gefahren. Zusätzlich kann das Fahrgestell 53 fixiert werden. Es kann ebenso eine Fixierung des Patientenstuhls 52 vorgesehen sein. Anschließend koppelt die Handhabungseinrichtung 7 an den Patientenstuhl 52 an. Nach dem Ankoppelvorgang können die Fixierungen von Patientenstuhl 52 und Fahrgestell 53 gelöst werden..

Fig. 13 zeigt schematisch den Ablauf eines Verfahrens zur automatisierten Positionierung eines Patienten. Im ersten Verfahrensschritt 70 wird die Position des Patiententransportmittels 1, 51 ermittelt und/oder das Patiententransportmittel 1, 51 wird in einer definierten Position angeordnet. Vorteilhaft wird das Patiententransportmittel 1, 51 zunächst in der definierten Position angeordnet, beispielsweise über eine Indexiereinrichtung, und anschließend wird die Position über Überwachungsmittel, beispielsweise ein optisches Erfassungssystem 24, 64, überprüft. Das optische Erfassungssystem 24, 64 kann jedoch auch dazu dienen, die Position des Patiententransportmittels 1, 51 zu bestimmen. Dabei ist auch eine Identifizierung der Patientenaufnahme 2, 52 vorgesehen.

Im zweiten Verfahrensschritt 71 wird das Patiententransportmittel 1, 51 in der Aufnahmeposition 18 fixiert. Hierzu kann entweder das Fahrgestell 3, 53 oder die Patientenliege 2 bzw. der Patientenstuhl 52 fixiert werden. Auch eine Fixierung von Fahrgestell 53 und Patientenaufnahme 2, 52 kann vorgesehen sein.

Im dritten Verfahrensschritt 72 wird die Patientenliege 2 bzw. der Patientenstuhl 52 an die Handhabungseinrichtung 7 angekoppelt.

Im vierten Verfahrensschritt 73 wird die Fixiereinrichtung 33 der Patientenliege 2 bzw. des Patientenstuhls 52 freigegeben. Anschließend wird die Patientenliege 2 bzw. der Patientenstuhl 52 im fünften Verfahrensschritt 74 in die Behandlungsposition 19 bewegt.

Im sechsten Verfahrensschritt 75 kann die Korrektur der Patientenliege 2 bzw. des Patientenstuhls 52 anhand von geometrischen Daten der identifizierten Patientenliege 2 bzw. des identifizierten Patientenstuhls 52 korrigiert werden. Hierzu wurde zuvor die Patientenliege 2 bzw. der Patientenstuhl 52 von dem optischen System identifiziert, insbesondere im ersten Verfahrensschritt 70.

Im siebten Verfahrensschritt 76 wird der Patient 13 der Behandlung, also einem Diagnose- und/oder einem Therapieverfahren, zugeführt. Dabei können auch aktuelle geometrische Daten der individuellen Patientenaufnahme 2, 52 ermittelt werden.

Im achten Verfahrensschritt 77 werden die gespeicherten geometrischen Daten der Patientenliege 2 bzw. des Patientenstuhls 52 korrigiert, soweit ein bildgebendes Verfahren bei der Behandlung zum Einsatz kam. Im folgenden neunten Verfahrensschritt 78 wird die Patientenaufnahme 2, 52 zurück in die Aufnahmeposition 18 bewegt.

Im zehnten Verfahrensschritt werden die Handhabungseinrichtung 7 und die Patientenaufnahme 2, 52 voneinander entkoppelt. Hierzu kann wieder eine Fixierung der Patientenaufnahme 2, 52 vorgesehen sein. Das Fahrgestell 3, 53 kann vom zweiten bis zum zehnten Verfahrensschritt fixiert bleiben.

## Patentansprüche

1. Einrichtung zur automatisierten Positionierung eines Patienten, wobei die Einrichtung ein bewegliches Patiententransportmittel (1, 51) mit einer Patientenaufnahme (2, 52) und eine Handhabungseinrichtung (7) umfasst, wobei eine Kopplungseinrichtung (29) zur Kopplung der Patientenaufnahme (2, 52) in einer Aufnahmeposition (18, 58) mit der Handhabungseinrichtung (7) vorgesehen ist,
**dadurch gekennzeichnet, dass** die Einrichtung Positionierungsmittel zur Festlegung und/oder Bestimmung der Position des Patiententransportmittels (1, 51) relativ zu der Handhabungseinrichtung (7) in entkoppeltem Zustand von Patientenaufnahme (2, 52) und Handhabungseinrichtung (7) besitzt.

2. Einrichtung nach Anspruch 1,
**dadurch gekennzeichnet, dass** die Positionierungsmittel mindestens eine Indexiereinrichtung zur Anordnung des Patiententransportmittels (1, 51) in der Aufnahmeposition (18, 58) umfassen.

3. Einrichtung nach Anspruch 2,
**dadurch gekennzeichnet, dass** die Indexiereinrichtung mindestens einen Indexierdorn (20, 60) umfasst, der mit einer Indexieraufnahme (21, 61) an dem Patiententransportmittel (1, 51) zusammenwirkt.

4. Einrichtung nach einem der Ansprüche 1 bis 3,
**dadurch gekennzeichnet, dass** die Positionierungsmittel ein optisches System mit einer optischen Marke (36, 66) an dem Patiententransportmittel (1, 51) und einem optischen Erfassungssystem (24, 64) zur Erfassung der Position der optischen Marke (36, 66) umfassen.

5. Einrichtung nach einem der Ansprüche 1 bis 4,
**dadurch gekennzeichnet, dass** die Positionierungsmittel ein Funksystem mit einer Funkmarke (45) an dem Patiententransportmittel (1) und einer Funkeinrichtung (46) zur Erfassung der Position der Funkmarke (45) umfassen.

6. Einrichtung nach Anspruch 4 oder 5,
**dadurch gekennzeichnet, dass** die Einrichtung mehrere Patiententransportmittel (1, 51) umfasst, wobei sich die Marken (36, 45, 66) der einzelnen Patiententransportmittels (1, 51) zur Identifizierung des einzelnen Patiententransportmittels (1, 51) unterscheiden.

7. Einrichtung nach einem der Ansprüche 1 bis 6,
**dadurch gekennzeichnet, dass** die Positionierungsmittel eine Vorpositionierungseinrichtung zur Vorpositionierung des Patiententransportmittels (1, 51) im Raum umfassen.

8. Einrichtung nach einem der Ansprüche 1 bis 7,
**dadurch gekennzeichnet, dass** mindestens eine Fixiereinrichtung (33) zur Fixierung des Patiententransportmittels (1) vorgesehen ist.

9. Einrichtung nach einem der Ansprüche 1 bis 8,
**dadurch gekennzeichnet, dass** eine Positionsüberwachungseinrichtung (39) zur Überwachung der Anordnung des Patiententransportmittels (1) in der Aufnahmeposition (18) vorgesehen ist.

10. Einrichtung nach einem der Ansprüche 1 bis 9,
**dadurch gekennzeichnet, dass** Überwachungsmittel zur Überwachung der Kopplung der Patientenaufnahme (2, 52) mit der Handhabungseinrichtung (7) vorgesehen sind.

11. Einrichtung nach einem der Ansprüche 1 bis 10,
**dadurch gekennzeichnet, dass** die Kopplungseinrichtung eine Toleranzausgleichseinrichtung (38) zum Ausgleich von Toleranzen zwischen der Handhabungseinrichtung (7) und der Patientenaufnahme (2, 52) aufweist.

12. Einrichtung nach einem der Ansprüche 1 bis 11,
**dadurch gekennzeichnet, dass** die Handhabungseinrichtung (7) ein Roboterarm ist, der mindestens eine vertikale Bewegungsachse (8) und mindestens zwei horizontale Bewegungsachsen (9, 10, 11, 12) aufweist.

13. Verfahren zur automatisierten Positionierung eines Patienten, mit einer Einrichtung zur automatisierten Positionierung des Patienten, wobei die Einrichtung ein bewegliches Patiententransportmittel (1, 51) mit einer Patientenaufnahme (2, 52) und eine Handhabungseinrichtung (7) umfasst, und wobei die Patientenaufnahme (2, 52) mit einer Kopplungseinrichtung mit der Handhabungseinrichtung (7) gekoppelt wird,
**dadurch gekennzeichnet, dass** das Patiententransportmittel (1, 51) vor der Kopplung mit Positionierungsmitteln im Raum positioniert und/oder die Position des Patiententransportmittels (1, 51) mit den Positionierungsmitteln bestimmt wird.

14. Verfahren nach Anspruch 13,
**dadurch gekennzeichnet, dass** die Patientenaufnahme (2) vor der Kopplung von einer Fixiereinrichtung (33) fixiert wird, und dass die Patientenaufnahme (2) nach der Kopplung mit der Handhabungseinrichtung (7) von der Fixiereinrichtung (33) freigegeben wird.

15. Verfahren nach Anspruch 13 oder 14,
**dadurch gekennzeichnet, dass** die Handhabungseinrichtung (7) Mittel zur Erfassung der im Betrieb an der Handhabungseinrichtung (7) wirkenden Kräfte aufweist, und dass die an der Handhabungseinrichtung (7) wirkenden Kräfte überwacht werden.

16. Verfahren nach einem der Ansprüche 13 bis 15,
**dadurch gekennzeichnet, dass** das Patiententransportmittel (1, 51) mindestens eine Marke (36, 45, 66) aufweist, und dass das Patiententransportmittel (1, 51) anhand der Marke (36, 45, 66) identifiziert und die Behandlungsposition (19) des Patienten (13) mit gespeicherten geometrischen Daten des identifizierten Patiententransportmittels (1, 51) korrigiert wird.
